# EUROPÄISCHE PATENTSCHRIFT

(11) **EP 0 324 745 B1**
(45) Veröffentlichungstag und Bekanntmachung des Hinweises auf die Patenterteilung: **11.01.1995**
(21) Anmeldenummer: 87905715.6
(22) Anmeldetag: 01.09.1987
(51) Int. Cl.: A61K 31/557

(54) **PROSTACYCLINDERIVATE ENTHALTENDE MITTEL FÜR DIE TOPISCHE ANWENDUNG**
TOPICAL AGENTS CONTAINING PROSTACYCLINE DERIVATIVES
AGENTS TOPIQUES CONTENANT DES DERIVES DE PROSTACYCLINE

(30) Priorität: 11.09.1986 DE 3631169
(43) Veröffentlichungstag der Anmeldung: 26.07.1989
(73) Patentinhaber: SCHERING AKTIENGESELLSCHAFT, 13342 Berlin (DE)
(72) Erfinder: EKERDT, Roland, Dr., D-1000 Berlin 28 (DE); RAPTIS, Georg, Dr., D-1000 Berlin 33 (DE); PAULS, Alfred, Dr., D-1000 Berlin 27 (DE)
(86) Internationale Anmeldenummer: DE8700401
(87) Internationale Veröffentlichungsnummer: WO8801867

(56) Entgegenhaltungen:
- EP-A- 0 011 591
- EP-A- 0 171 992
- WO-A-86/00808
- Chemical Abstracts, volume 101, No. 21, 19 Nov. 1984, (Columbus, 0hio, US), R. Ekerdt et al.: "Blood supply can be increased in rabbit skin by a stable prostacyclin derivative without protentiation of plasma extravasation", p. 152, abstract 184795w
- Dorland's Illustrated Medical Dictionary, 26th ed., W.B. Saunders Co., pp. 1417-1418

## Beschreibung

Die vorliegende Erfindung betrifft eine neue Verwendung von Prostacyclinderivaten zur Herstellung von Mitteln für die topische Anwendung zur Behandlung der Altershaut, von Ulcerationen durch arterielle Verschlußkrankheiten oder von schwer heilenden Wunden.

Ulcerationen treten auf, wenn der Blutfluß in den befallenen Körperregionen durch mechanische oder krankhafte Veränderungen der Blutgefäße verringert oder ganz unterbunden ist. So sind bei Veränderungen der Arterien, wie sie bei M. Raynaud und obliteriender Artheriosklerose beschrieben sind, Ulcerationen der Extremitäten häufig. Von den bekannten medikamentösen Behandlungen für M. Raynaud und obliterierende Artheriosklerose haben die mit Prostaglandinen, in den letzten Jahren sehr großes Interesse hervorgerufen. So sind PGE ₁ und Prostacyclin (PGI₂) erfolgreich bei diesen Indikationen eingesetzt worden, allerdings - bedingt durch die geringe Stabilität dieser natürlichen Prostaglandine - stets als Dauerinfusion (R.J. Gryglewski et al., The Lancet, 1111 (1979)).

Es wird berichtet, daß unter diesen Bedingungen Ulcerationen vollständig abheilen. Da vasodilatierende Pharmaka mit den unterschiedlichsten Wirkprinzipien speziell bei Artheriosklerose unwirksam waren, ist es nicht sehr wahrscheinlich, daß die positive Beeinflussung der Ulcerationen nur auf die vasodilatierene Wirkung der eingesetzten Prostaglandine zurückzuführen ist.

Prostacyclin hat neben seiner relaxierenden Wirkung auf die Gefäßmuskulatur unter anderem eine Blutplättchenaggregation-hemmende Wirkung, beschleunigt die Desaggregation von Thromben und verbessert die Fließeigenschaften des Blutes über die Verformbarkeit von Erythrozyten, aber auf eine direkte Korrelation zwischen Wirkung von PGI₂ und Abheilen von Ulcerationen wurde bisher nicht hingewiesen.

J-E. Birnbaum et al. (Prostaglandins, 23, 185 (1982)) empfehlen die topische Anwendung von 16-Vinylprostaglandinen zur Blutdrucksenkung, um die negativen Begleiterscheinungen einer oralen oder intravenösen Applikation wie z.B. Belastung des Gastrointestinaltraktes auszuschalten.

Häufig wird gegen eine topische Anwendung von Prostaglandin-Derivaten eingewendet, daß die topisch applizierte Substanz größere tiefer liegende Gefäße nicht erreichen kann und deshalb verfehlt sei. R.J. Gryglewski et al. (l.c.) berichten, daß nach i.v. Infusion von PGI₂ die in einem Angiogramm sichtbaren Läsionen in den Hauptarterien von Patienten mit A.obliterans unverändert waren, obwohl eine positive Beeinflussung des klinischen Bildes vorlag. Diese Beobachtung spricht nach Ansicht der Autoren für eine Einwirkung von PGI₂ auf kleinere Arterien, die zum Abheilen von Ulcerationen führt.

Aus WO86/00808 sind Prostacyclinderivat-haltige Mittel mit zytoprotektiver Wirkung, an Organen bekannt. Ekerdt et al. beschreiben in British J. of Dermatology 111, 144 (1984) eine proinflammatorische Nebenwirkung für Iloprost bei topischer Anwendung.

Es wurde nun die topische Verwendung von Prostacyclinderivaten der allgemeinen Formel I 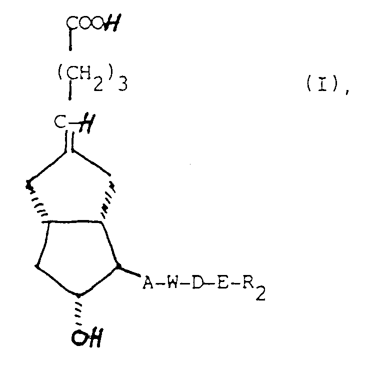
worin
- A: eine trans-CH=CH- oder -C≡C-Gruppe,
- W: eine Hydroxymethylengruppe, wobei die OH-Gruppe α- oder β-ständig ist,
- D: eine geradkettige oder verzweigte gesättigte Alkylengruppe mit 1-5 C-Atomen,
- E: eine -C≡C-Bindung,
- R₂: eine gerad- oder verzweigtkettige, gesättigte Alkylgruppe mit 1-7 C-Atomen,
und deren Salze mit physiologisch verträglichen Basen bedeuten, zur Herstellung von Mittel zur Behandlung von Altershaut, Ulcerationen durch arterielle Verschlußkrankheiten und schwer heilenden Wunden gefunden.

Als Alkylgruppe R₂ kommen gerad- und verzweigtkettige, gesättigte Alkylreste mit 1-7 C-Atomen in Frage. Beispielsweise genannt seien Methyl, Äthyl-, Propyl-, Butyl- und Isobutyl, tert.-Butyl, Pentyl-, Hexyl-, Heptyl.

Als Alkylgruppe D kommen geradkettige oder verzweigtkettige, gesättigte mit bis zu 5 C-Atomen in Betracht. Beispielsweise genannt seien Methylen, Äthylen, 1.2-Propylen, Äthyläthylen, Trimethylen, Tetramethylen, Pentamethylen, 1-Methyl-tetramethylen, 1-Methyl-trimethylen.

Zur Salzbildung mit den freien Säuren sind anorganische und organische Basen geeignet, wie sie dem Fachmann zur Bildung physiologisch verträglicher Salze bekannt sind. Beispielsweise seinen genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Herstellung der Verbindungen der Formel I wird detailliert in EP 2234 und EP 11591 beschrieben.

In EP 11591 werden für die Carbacyclinderivate der Formel folgende pharmakologische Eigenschaften beschrieben:
Senkung des peripheren arteriellen und koronaren vaskulären Widerstandes, Inhibierung der Thrombozytenaggreation und Auflösung von Plättchenthromben, myocardiale Zytoprotektion; Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonalen vaskulären Widerstandes und des pulmonalen Blutdruckes, Förderung der Nierendurchblutung, Anwendung an Stelle von Heparin oder als Adjuvans bei der Dialyse oder Hämofiltration, Konservierung von Blutplasmakonsernen, besonders von Blutplättchenkonserven, Inhibierung von Geburtswehen, Behandlung von Schwangerschaftstoxikose, Erhöhung der zerebralen Durchblutung und Antiproliferation.

In EP 11591 werden parenterale Verabreichungsformen (injizierbare wässrige oder ölige Lösungen) genannt. Topische Verabreichungsformen waren dagegen ausgenommen. Es zeigt sich nun überraschend, daß man Ulcerationen in den befallenen Körperregionen oder M. Raynaud auch nach topischer Anwendung der oben genannten Prostacyclinderivate erfolgreich behandeln kann, ohne dabei den übrigen Organismus zu belasten, wie es bei der Behandlung mit Infusion, i.m.-bzw. i.v.-Injektion die Regel war.

Da die topische Anwendung von Prostacyclinderivaten der Formel I eine lokal begrenzte, in Starke und Dauer steuerbare Hyperämie erzeugt, ist sie besonders geeignet, gezielt in ischämischen Hautarealen eingesetzt zu werden. Weiterhin kann die topische Anwendung von Prostacyclinderivaten der Formel I in der erkrankten Körperregion ein optimales Verhältnis zwischen lokal verfügbarer Konzentration und minimaler Belastung durch Nebenwirkungen im Gesamtorganismus ergeben.

Es wurde der Einfluß der Prostacyclinderivate der Formel I auf die Blutversorgung der Haut untersucht. Mit dieser Information können Aussagen über die Verfügbarkeit der untersuchten Verbindungen in der Haut gemacht werden.

Weiterhin wurden alle wichtigen systemischen und lokalen Nebenwirkungen für die topische Anwendung von Prostacyclinderivaten der Formel I, nämlich Beeinflussung des Blutdruckes und der Herzfrequenz sowie proinflammatorische Wirkung im entzündeten Gewebe, im Tierexperiment bestimmt und eingegrenzt.

Zur Behandlung der Altershaut wird z. B. Iloprost in niedriger Konzentratration topisch appliziert, so daß jede Rötung vermieden wird, aber noch nachweislich minimale Temperaturerhöhungen der bestrichenen Hautpartien auftreten (als Nachweis einer verbesserten Durchblutung). Dazu bedient man sich temperatursensibler flüssiger Kristalle.

Die dermale Applikation von Prostacyclinderivaten der Formel I erweist sich bei der Therapie folgender Erkrankungen als vorteilhaft:
Altershaut
Ulcus cruris
Dekubitalulcus
schwer heilende Wunden.

Die Erfindung betrifft damit auch Arzneimittel auf Basis der Verbindungen der Formel I und üblicher Hilfs- und Trägerstoffe. Die erfindungsgemäßen Wirkstoffe sollen in Verbindung mit den in der Galenik bekannten und üblichen Hilfsstoffen, z.B. zur Herstellung von Dermatika dienen. Diese Dermatika können in flüssiger, halbfester oder fester Form vorliegen.

Die Formulierung wird auf die erkrankte Hautstelle so aufgetragen, daß die Flächenkonzentration des Wirkstoffes zwischen 0,01 und 50 µg/cm² auf der Haut beträgt. Das so behandelte Hautareal wird besser durchblutet, was durch Rötung oder lokalen Temperaturanstieg registriert werden kann. Diese Wirkung ist in Stärke und Dauer von der Dosis abhängig. Eine systemische Wirkung auf die Blutgefäße und auf die Thrombozyten wird nicht beobachtet. Ebenso treten bis zu einer Dosierung von 500 µg pro Mensch keine Verträglichkeitsprobleme auf. Die behandelten Patienten können während der Behandlung ihrer gewohnten Tätigkeit nachgehen.

### Beispiele

### Beispiel 1

34,5 µg Iloprost in 0,6 ml Wasser gelöst wruden einem Probanden (85 kg Körpergewicht) auf 100 cm² seiner Rückenhaut aufgetragen. Das entspricht einer Flächenkonzentration von 0,35 µg/cm² an Iloprost.

Nach 15 Minuten wurde eine deutliche Rötung der behandelten Fläche festgestellt. Die lokale Temperatur der behandelten Fläche lag im Vergleich zu der Temperatur der benachbarten Haut um 2 °C höher. 72 Stunden nach der Applikation war die Rötung wieder abgeklungen.

Es wurden dabei keine lokalen unangenehmen Reizungen wie Ödeme, Juckreiz oder Brennen festgestellt. Bei der ständigen Kontrolle von Blutdruck, Herzfrequenz, Körpertemperatur, Atemfrequenz und Thrombozytenaggregation (TA) vor Applikation und 0,25; 0,50; 1; 1,5; 2; 4; 6; 8; 24; 36; 48 und 60 Stunden nach Applikation wurden keine Effekte beobachtet, die auf eine systemische Wirkung zurückzuführen wären. Das Allgemeinbefinden des Probanden war daher zu keinem Zeitpunkt beeinträchtigt.

### Beispiel 2

Ein Probend (74 kg Körpergewicht) erhielt 10 µg Iloprost in 0,2 ml wäßriger Lösung auf 30 cm² Hautfläche des Rückens verteilt. Die Hornschicht der behandelten Hautfläche war zuvor durch mehrmaliges Kleben und Abreissen eines Tesafilms entfernt worden. Analog Beispiel 1 wurden Blutdruck, Herzfrequenz, Körpertemperatur, Atemfrequenz und TA gemessen. Es wurde lediglich eine stärkere Rötung der gestrippten und behandelten sowie der benachbarten Haut registriert. Dieser Effekt dauerte ca. 3 Tage.

### Beispiel 3

Die topische Anwendung von Iloprost am Kaninchen erzeugt eine lokal begrenzte, in Stärke und Dauer steuerbare Förderung der Hautdurchblutung. Die niedrigste effektive Dosis von 70 ng/cm² auf intakte Haut erhöht die Hautdurchblutung um 300 % und hält länger als 4 h an.

Da man bei intracutaner Injektion die Durchblutung auf einer Hautflache von ca. 1 cm² erhöht, ergibt der Vergleich mit der niedrigsten effektiven Dosis bei intracutaner Applikation (0.3 - 0.5 ng) ein 50-100faches empfindlicheres Ansprechen der Durchblutung bei Umgehung des Stratum corneums.

Durch Vergleich der Dosisbereiche von Hautdurchblutungssteigerung auf intakter Haut und Ödem-steigernder Wirkung auf entzündeter Haut erhält man den Dosisbereich (70-700 ng/cm²), bei dem die Hautdurchblutung noch gefördert wird, ohne daß eine proinflammatorische Wirkung in entzündetem Gewebe aufgelöst wird. Die Dissoziation von Hautdurchblutung und Ödem-steigernder Wirkung wird beim Vergleich der jeweils niedrigsten wirksamen Dosierung deutlich. Erst bei der hundertfachen (7000 ng/cm² für Histamin) bzw. 10fachen (700 ng/cm² für Crotonöl) durchblutungssteigernden Dosis wurde eine proinflammatorische Wirkung von Iloprost meßbar.

Die Resorption von Iloprost nach topischer Anwendung und die damit verquickte Beeinflussung von Blutdruck und Herzfrequenz hängt von der aufgetragenen Gesamtmenge und der Beschaffenheit der Haut ab. Selbst bei großflächiger Behandlung (ca. 5 % der Gesamtkörperoberfläche) mit Iloprost in einer für die Förderung der Hautdurchblutung relevanten Dosierung (72-720 ng/cm²) konnte keine Beeinflussung von Blutdruck und Herzfrequenz gemessen werden.

Diese Ergebnisse wurden sowohl für intakte als auch für geschädigte Haut erzielt. 7200 ng/cm² Iloprost bewirkte nur bei geschädigter Haut eine Senkung des Blutdruckes (50 % vom Ausgangswert). Systemische Nebenwirkungen sind für die angestrebten (70 ng/cm²) Dosierungen nicht zu erwarten.

Bei Beurteilung der gemessenen Plasmaspiegel muß man berücksichtigen, daß die Konzentration an Wirksübstanz wegen der Kreuzreaktion des Radioimmunassays mit Metaboliten von Iloprost niedriger als der gemessene Wert liegt. Da die Plasmaspiegel nach Applikation von 72 ng/cm² auf intakte Haut unterhalb der Nachweisgrenze des Assays lagen (100 pg/ml Plasma), kann eine systemische Thrombozytenaggregationshemmung für die angestrebte Dosierung (70 ng/cm²) ebenfalls ausgeschlossen werden.

## Patentansprüche

1. Topische Verwendung von Prostacyclinderivaten der Formel I 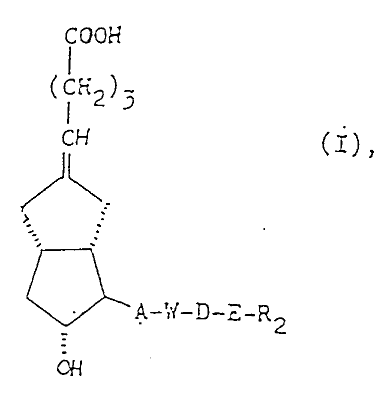 worin
A eine trans -CH=CH- oder -C≡C-Gruppe,
W eine Hydroxymethylengruppe, wobei die OH-Gruppe α- oder β-ständig ist,
D eine geradkettige oder verzweigte gesättigte Alkylengruppe mit 1-5 C-Atomen,
E eine -C≡C-Bindung
R₂ eine gerad- oder verzweigtkettige, gesättigte Alkylgruppe mit 1-7 C-Atomen und deren Salze mit physiologisch verträglichen Basen bedeuten,
zur Herstellung von Mitteln zur Behandlung von Altershaut, Ulcerationen durch arterielle Verschlußkrankheiten und schwer heilenden Wunden.

2. Topische Verwendung vom Iloprost nach Anspruch 1.

## Claims

1. Topical use of prostacylin derivatives of formula I 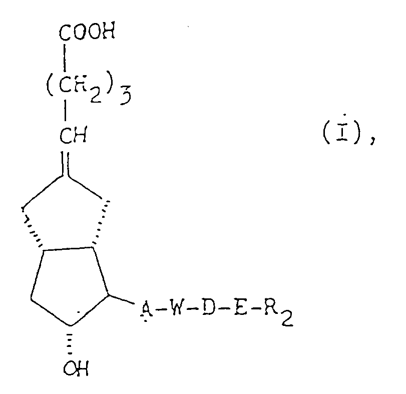 wherein
A is a trans -CH=CH- or -C≡C- group,
W is a hydroxymethylene group, the OH group being in the α- or the β-configuration,
D is a straight-chained or branched saturated alkylene group having from 1 to 5 carbon atoms,
E is a -C≡C- bond, and
R₂ is a straight-chained or branched-chained saturated alkyl group having from 1 to 7 carbon atoms,
and the salts thereof with physiologically tolerable bases, for the preparation of compositions for the treatment of the skin of old age, ulcerations caused by occlusive arterial diseases, and wounds that are slow to heal.

2. Topical use of iloprost according to claim 1.

## Revendications

1. Emploi topique (local) de prostacyclines de formule générale I ci-dessous : 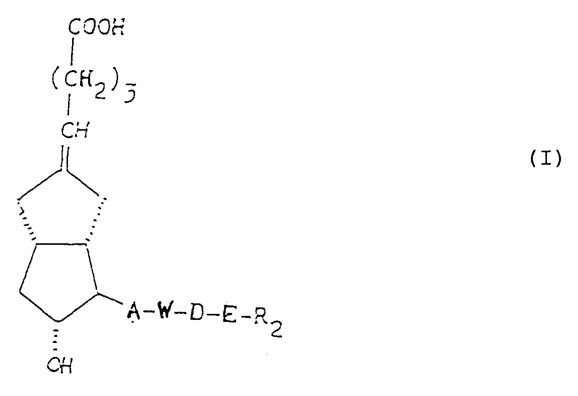 dans laquelle
A désigne un groupe trans-CH=CH- ou -C≡C,
W un groupe hydroxyméthylène dont l'OH est à la position α ou β,
D un alkylène saturé, linéaire ou ramifié, pouvant avoir de 1 à 5 atomes de carbone,
E une liaison -C≡C-, et
R₂ un alkyle saturé, linéaire ou ramifié, pouvant avoir de 1 à 7 atomes de carbone,
ou de sels de ces prostacyclines formés avec des bases physiologiquement compatibles et tolérées,
pour en préparer des produits en vue du traitement du vieillissement de la peau, d'ulcérations produites par des maladies obturant les artères, ainsi que de blessures difficiles à faire cicatriser et à guérir.

2. Emploi topique d'iloprost selon la revendication 1.
